# EUROPEAN PATENT SPECIFICATION

(11) **EP 3 534 973 B1**
(45) Date of publication and mention of the grant of the patent: **14.12.2022**
(21) Application number: 17818031.1
(22) Date of filing: 02.11.2017
(51) Int. Cl.: A61L 15/26, A61L 15/28, A61L 15/44

(54) **METHOD FOR PRODUCING A NANOFIBER LAYER AND A TEXTILE COMPOSITE COMPRISING THE NANOFIBER LAYER**
VERFAHREN ZUR HERSTELLUNG EINER NANOFASERSCHICHT UND EINER TEXTILER VERBUNDSTOFF MIT DER NANOFASERSCHICHT
PROCÉDÉ DE FABRICATION D'UNE COUCHE DE NANOFIBRES ET COMPOSITE TEXTILE COMPRENANT LA COUCHE DE NANOFIBRES

(30) Priority: 03.11.2016 CZ 20160688
(43) Date of publication of application: 11.09.2019
(73) Proprietor: Nano Medical, S.r.o., 140 00 Praha 4 (CZ); Univerzita Jana Evangelisty Purkyne V Ústí Nad Labem, 400 96 Ústí nad Labem (CZ)
(72) Inventor: MUNZAROVÁ, Marcela, 468 22 Zelezný Brod (CZ); MALY, Jan, 412 01 Litomerice (CZ); HOCELÍKOVÁ, Lucie, 436 01 Litvínov (CZ); POUSTKA, David, 360 01 Karlovy Vary (CZ)
(74) Representative: Markes, Libor
(86) International application number: PCT/CZ2017/000070
(87) International publication number: WO 2018/082721

(56) References cited:
- US-A1- 2014 043 236
- DATABASE WPI Week 201657 Thomson Scientific, London, GB; AN 2016-28998K XP002778036, & CN 105 536 577 A (UNIV DONGHUA) 4 May 2016 (2016-05-04)

## Description

### Technical Field

The invention relates to a method for producing a nanofiber layer based on chitosan in a solution with polyethylene oxide (PEO), designed as a textile composite for wound dressing, as well as a primary wound cover containing a nanofiber layer, material composition of the nanofiber layer, and the connection of the nanofiber layer with an absorbent or mechanical carrier.

### Background Art

At present, experts are engaged in the production of nanofibers from biocompatible materials, whose properties imitate actual tissues and which the body is then able to absorb to a greater or lesser extent. There are various ways of using the nanofiber layer and its saturation with various therapeutic agents, as well as various ways of linking this layer with the carrier.

Document TW 200740472 describes the method of manufacturing a wound dressing fabric comprising a nanofiber layer formed by electrostatic spinning. The acidic spinning solution contains collagen, chitosan and polyethylene oxide (PEO). The paper does not mention the steps that would lead to the stabilization of the nanofiber layer in a humid environment.

KR 20150125881 also describes a process for making a nonwoven fabric from nanofibers. The spinning solution contains water-soluble chitosan and PEO. The procedure does not include any steps that would lead to the structural stabilization of the nanofiber layer.

US 20130150763 relates to a wound dressing fabric composed of three nanofiber layers. The first, external hydrophobic layer consisting of nanofibers from biocompatible polymers has a supportive function. The second, middle, hydrophilic layer crosslinked by genipin serves as a reservoir of biologically active substances, formed from a chitosan and PEO solution in one of the versions. Genipin is added to the spinning solution at a chitosan ratio of 0.5 to 10 wt%. The crosslinking of chitosan nanofibers by genipin inhibits the initial release of herbal extracts and prolongs their release time. The third, hydrophilic layer comprising non-crosslinked chitosan provides the dressing's contact with the wound. Various additives are added to these layers in the exemplary versions, which influence the speed of release for herbal extracts.

A disadvantage of this solution is the cost associated with the use of genipin as a crosslinking agent.

CN 105536577 defines a method for preparing a chitosan nanofiber-based composite *filter membrane for removal of acid dyes from wastewater. The preparation comprises 5 steps:*

*Step 1: Dissolving a polymer material, for example polyether, in a solvent to prepare an electrospinning solution; Step 2*: *Chitosan and a spin-spun polymer, for example polyether, are dissolved in a solvent, whereas a certain amount of ultraviolet lightcrosslinkable monomer, for example TEGMA, is added, and the mixture is stirred to obtain an electrostatic spray solution; Step 3: Electrospinning the solution obtained in Step 1 to obtain a nanofiber nonwoven fabric support layer; Step 4: Spraying the electrostatic spray solution obtained in Step 2 by electrostatic spraying on the obtained nanofiber fabric. This results in formation of a functional barrier layer on the surface of the nanofabric nonwowen fabric support layer and formation of a composite film by the two layers together; Step 5: The composite film obtained in Step 4 is subjected to hot dressing and thawing treatment, and then subjected to ultraviolet light crosslinking.*

*This process results in formation of a membrane consisting of two layers: a functional barrier layer on nanofiber nonwoven fabric, the functional layer being constituted by colloid chitosan* particles.

Document US 2014/0043236 A1 discloses textiles for wound dressing comprising chitosan nanofibers. A mixture of chitosan and PEO is electrospun on a substrate.The obtained layer of fibers is then stabilized by neutralization with a base (NaOH).

The objective of the present invention is to provide a method for producing a stabilized nanofiber layer based on chitosan without the use of expensive chemical crosslinkers, which in addition allows crosslinking as required at any time after the production of the nanofiber layer.

### Disclosure of the invention

*The objective of the invention is to provide a nanofiber layer made from chitosan or a mixture of chitosan*/*gelatine according to claim 1, wherein the chitosan is chemically modified* by *methacrylate groups.*

*Another* objective of the present invention is to provide a method for producing a nanofiber layer based on chitosan in a solution with polyethylene oxide (PEO) designed for a textile composite for wound dressing. The method comprises the following steps:
- mixing the chitosan monomer with a methacrylate-containing agent in an aqueous medium,
- subjecting the mixture to filtration, washing it with H₂O and drying it without access to light,
- crushing into powder the resulting product, which is soluble solid phase modified chitosan,
- subsequently preparing a spinning mixture by dissolving the modified chitosan and polyethylene oxide (PEO) in an aqueous acetic acid solution,
- applying the spinning solution prepared in this way on a suitably selected substrate,
- crosslinking the nanofiber layer material.

The most advantageous version of the method comprises the following steps:
- mixing a chitosan monomer with -NH₂ group at 80% deacetylation dissolved in an aqueous environment at a ratio of 5 g chitosan to 10 ml water with glycidyl methacrylate (GMA) at a ratio of 2 mol chitosan to 1 mol GMA, and mixing the mixture for 24 hrs.,
- subjecting the mixture to filtration, washing it by H₂O and drying it for two days without access to light,
- crushing the resulting product into powder, which is solid phase modified chitosan soluble in an acidic aqueous medium,
- subsequently preparing a spinning mixture by dissolving the modified chitosan and PEO in an aqueous acetic acid solution at the volume ratio of water to acetic acid 2 : 1 in such a way that the resultant solution contains 2 wt% to 10 wt% chitosan and 0.1wt% to 0.3 wt% PEO,
- applying the spinning solution prepared in this way on a suitably selected substrate,
- crosslinking the nanofiber layer material.

Crosslinking can be performed by exposing the nanofiber layer to UV radiation.

In another version of the invention, the method can comprise the following steps:
- adding gelatine to the modified chitosan and PEO solution in such a way that the resultant solution contains 4 wt% to 9 wt% chitosan, 0.1 wt% to 0.3 wt% PEO and 0.5 wt% to 5.0 wt% gelatine,
- crosslinking the nanofiber layer material by exposure to UV radiation.

Crosslinking can be performed by exposing the nanofiber layer to a temperature of 130°C for about 10 min.

A crosslinking agent tetramethylene glycol dimethacrylate (TEGMA) can be added to the spinning mixture.

The nanofiber layer can be connected, through a lamination process, to another textile composite layer using adhesives or non-adhesive technologies.

The substrate used in the production of nanofibers can be removed during the lamination process or before the composite is applied.

The nanofiber layer can be provided with a protective envelope, which is removed before the wound cover is applied.

The nanofiber layer is standardly infused with bioactive additives such as antimicrobial substances, analgesic substances, antibiotics, chemotherapeutics or enzymes.

Therefore, the objective of the invention is to prepare a nanofiber layer from chitosan or a mixture of chitosan/gelatine. Before preparing the spinning solution, the used chitosan (raw material) is chemically modified according to methacrylate groups. The modification of chitosan allows the use of UV in order to ensure the stability of the nanofiber structure in an aqueous environment after the production of the nanofiber structure. A nanofiber structure made from modified chitosan and subsequently stabilized by UV radiation is used to make a wound cover.

The method of manufacturing a wound cover consists of connecting a nanofiber structure with a textile carrier using lamination technology with or without the use of adhesives.

Preparation of nanofiber layers:
The nanofiber layer is prepared from a chitosan, PEO and optionally gelatine solution dissolved in acetic acid and water. The advantage of using this mixture lies in the combination of the positive properties of polymers related to wound healing. Chitosan and gelatine are biodegradable in a number of days. When used to heal wounds, they are partially decomposed.

The nanofiber layer made from chitosan or chitosan/gelatine mixture is unstable in an aqueous environment. In order to stabilize it, the thermal or chemical crosslinking of chitosan is required.

Thermal stabilization takes place at a temperature of about 130°C for about 10 minutes. The thermal crosslinking of chitosan will also stabilize the gelatine for the required time, i.e. for about the 24 hours necessary to start the tissue granulation and re-epithelisation process.

Chemical crosslinking takes place through the chemical modification of the chitosan polymer according to methacrylate groups and the subsequent crosslinking initiated by UV radiation is applied after the production of nanofibers.

The synthesis of the modified chitosan takes place at neutral pH, the modified polymer well soluble in the spinning solution (60% acetic acid) and possible to produce nanofibers from it.

**Table 1**

| Loading of chitosan g/100ml/molar concentration of monomer with -NH₂ group at 80% deacetylation | Volume of GMA up to 100ml dH₂O/resulting concentration | Theoretical molar ratio chitosan monomer/GMA | Experimentally Specified degree of modification of NH₂ groups |
|---|---|---|---|
| 5 g (164 mM/monomer) | 1.084 ml (82 mM) | 2/1 | 18% |

Table 1 contains an example of a chitosan modification process (KitoZyme) by glycidyl methacrylate at neutral pH. The molar concentration of chitosan monomer with an amino group is derived from an 80% representation of chitosan in the product and an 80% chitosan deacetylation level.

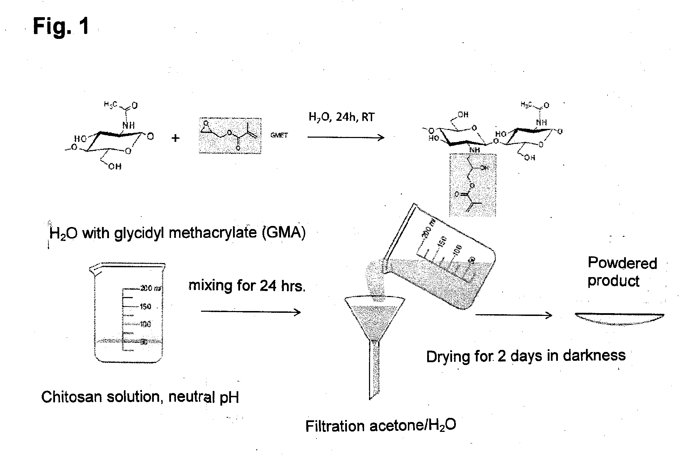

The chitosan modification process at a neutral pH is evident from Fig. 1.

The basis of light-activated crosslinking is the formation of chemical reactive groups, the so-called photoinitiator radicals through the absorption of UV radiation, the subsequent cleavage of the double bonds of the methyl methacrylate groups of the chitosan/TEGMA crosslinker with the formation of new radicals. Their repeated reaction with other methyl methacrylate groups results in the covalent crosslinking of chitosan and TEGMA crosslinker (or only chitosan, if TEGMA is not part of the spinning mixture). This cross-linking mechanism is described in detail in the literature dealing with the development of biocompatible hydrogels, such as, for example, hydrogel based on alginate modified by methyl methacrylate. From the point of view of the reaction mechanism, it is similar to the mechanism we anticipate for modified chitosan.

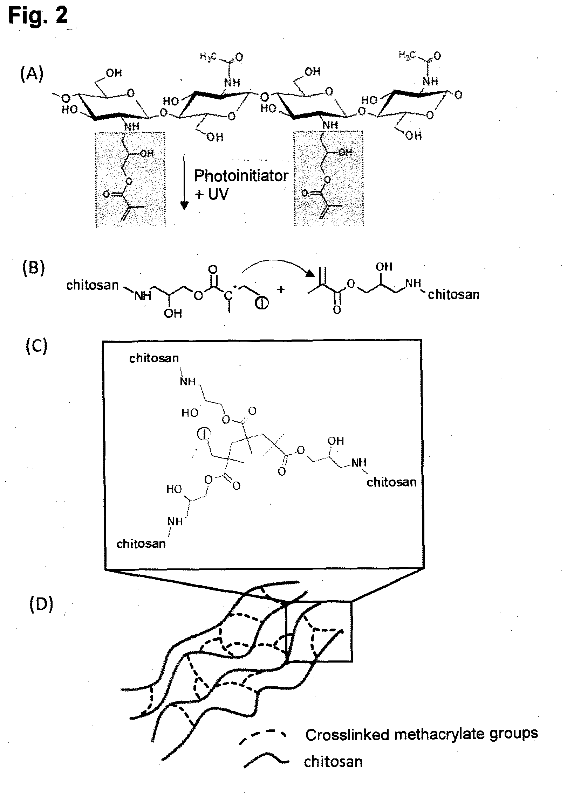

Figure 2 illustrates a possible mechanism of the light-activated cross-linking of chitosan modified by methacrylate: (A) chitosan modified by methacrylate groups; (B) the mechanism of reactive radical formation by UV radiation in the presence of a photoinitiator; (C) the formation of covalent bonds and crosslinking of chitosan; (D) the predicted structure of the resulting nanofiber.

The combination of polymers creates fibres, which have the ability to absorb fluids, resulting in their gelatinization - this creates a humid environment suitable for wound healing and suitable pH in the wound (the pH value of the material is 7.4).

The structure of the nanofiber layer imitates the structure of the extracellular matrix of the tissue, which means that it provides cells with good conditions for granulation (taking place from the edge of the wound towards the wound centre) and subsequent re-epithelisation through skin cells (fibroblasts). The stability of the nanomaterial in a humid environment is 24 to 48 hours. After 72 hours, it is possible to detect the residues of biodegradable material at the application site.

### Brief Description of the Drawings

The invention will be further illustrated by means of drawings, where:
- Fig. 1: shows a chitosan modification scheme at neutral pH;
- Fig. 2: shows a mechanism of light-activated cross-linking of chitosan modified by methacrylate: (A) chitosan modified by methacrylate groups; (B) the mechanism of reactive radical formation by UV radiation in the presence of a photoinitiator; (C) the formation of covalent bonds and the crosslinking of chitosan; (D) the predicted structure of the resulting nanofiber.

### Examples of carrying out the invention

### Example 1

Laminate structure: cover layer (substrate) nonwoven fabric spunbond 20-30 g/m² A nanofiber layer is prepared from chitosan chemically modified by methacrylate groups, whose structural stability is ensured by the UV crosslinking after the production of the nanofiber layer.

### Example 2

Laminate structure: cover layer (substrate) nonwoven fabric spunbond 20-30 g/m² A nanofiber layer is made from a solution containing 2 wt% to 10 wt% chitosan and 0.1 wt% to 0.3 wt% PEO, while acetic acid and water are added at a 2:1 ratio to complete the solution.

### Example 3

Laminate structure: cover layer (substrate) nonwoven fabric spunbond 20-30 g/m² A nanofiber layer is made from a solution containing 4 wt% to 9 wt% chitosan, 0.1 wt% to 0.3 wt% PEO and 0.5 wt% to 5 et% gelatine, while acetic acid and water are added at a 2:1 ratio to complete the solution.

In Examples 2 and 3, the nanofiber layer is bonded to an absorbent layer through an adhesive approved for use in medical devices. This adhesive may be a powder glue (application 3-10 g/m²) or a fusible mesh - e.g. PO 4605 (10-20 g/m²) or a hot-melt glue.

Another option for bonding layers is a non-adhesive bonding, e.g. ultrasonic or thermal bonding.

The substrate, which is used to make the nanofiber layer, can be part of the product always removed before application. In other applications, the substrate can be removed during the lamination process and the product is provided with a protective envelope removed prior to application.

The nanofiber layer is applied directly to the wound, the absorbent layer used in Examples 2 and 3 located in the direction out of the wound. The cover is usually fixed at the application spot by means of a secondary cover - adhesive foil or dressing materials. The cover can also be provided with an adhesive system on the edges and on the outside, which ensures its easy application and fixation at the desired location.

The nanofiber layer may contain bioactive additives such as antimicrobial substances, analgetic substances, antibiotics, chemotherapeutics, enzymes, etc.

## Claims

1. Nanofibers made of chitosan or a mixture of chitosan/gelatine, wherein the chitosan is chemically modified by methacrylate groups.

2. A method for producing a nanofiber layer of chitosan nanofibers according to claim 1 in a polyethylene oxide (PEO) solution designed for a textile composite for wound dressing, **characterized in** the following steps:
- mixing a chitosan monomer with a methacrylate-containing agent in an aqueous medium,
- subjecting the mixture to filtration, washing it with H₂O and drying it without access to light,
- crushing the resulting product into powder, a soluble solid phase modified chitosan,
- subsequently preparing a spinning mixture by dissolving the modified chitosan and polyethylene oxide (PEO) in an aqueous acetic acid solution,
- applying the spinning solution prepared in this way on a suitably selected substrate,
- crosslinking the nanofiber layer material.

3. The method according to Claim 2, **characterised in** the following steps:
- mixing a chitosan monomer with a -NH₂ group at 80% deacetylation dissolved in an aqueous environment at a ratio of 5 g chitosan to 10 ml water with glycidyl methacrylate (GMA) at a ratio of 2 mol chitosan to 1 mol GMA, and blending the mixture for 24 hrs.,
- subjecting the mixture to filtration, washing it by H₂O and drying it for two days without access to light,
- crushing the resulting product into powder, a solid phase modified chitosan soluble in an acidic aqueous medium,
- subsequently preparing a spinning mixture by dissolving the modified chitosan and PEO in an aqueous acetic acid solution at the volume ratio of water to acetic acid 2:1 in such a way that the resultant solution contains 2 wt% to 10 wt% chitosan and 0.1wt% to 0.3 wt% PEO,
- applying the spinning solution prepared in this way on a suitably selected substrate,
- crosslinking the nanofiber layer material.

4. The method according to Claim 2 or 3, **characterised in that the** crosslinking is performed by exposing the nanofiber layer to UV radiation.

5. The method according to Claim 2 or 3, **characterised in** the following steps:
- adding gelatine to the solution of modified chitosan and PEO in such a way that the resultant solution contains 4 wt% to 9 wt% chitosan, 0.1 wt% to 0.3 wt% PEO and 0.5 wt% to 5.0 wt% gelatine,
- crosslinking the nanofiber layer material by exposing to UV radiation.

6. The method according to Claim 5, **characterised in that the** crosslinking is performed by exposing the nanofiber layer to a temperature of 130°C for about 10 min.

7. The method according to Claims 2 to 6, **characterised in that** the crosslinking agent tetramethylene glycol dimethacrylate (TEGMA) is added to the spinning mixture.

8. The method according to Claims 2 to 7, **characterised in that** the nanofiber layer is connected, through a lamination process, to another textile composite layer using adhesives or non-adhesive technologies.

9. The method according to Claim 8, **characterised in that** the substrate used in the production of nanofibers is removed during the lamination process or before the composite is applied.

10. The method according to Claim 9, **characterised in that** the nanofiber layer is provided with a protective envelope, which is removed before the wound cover is applied.

## Patentansprüche

1. Aus Chitosan oder aus einem Chitosan/Gelatine-Gemisch hergestellte Nanofiber, wobei Chitosan durch Methakrylat-Gruppen modifiziert ist.

2. Herstellungsverfahren einer Nanofaser-Membran aus Chitosanfasern nach Anspruch 1 in einer Polyethylenoxid-Lösung (PEO) zur Bildung eines Textil-Composits bestimmt für Wundenbedeckung, **gekennzeichnet durch** die folgenden Schritte:
- Mischungsvorbereitung eines Chitosan-Monomers mit einem Methakrylat enthaltenden Mittel in einem aquatischen Medium,
- Filtration, Durchwaschen mit H₂O und Trocknen des Gemisches ohne Lichteinfall,
- Stoßen des Endprodukts, des löslichen modifizierten Chitosans in fester Phase, zu Pulver,
- nachfolgende Vorbereitung eines Gemisches zum Verspinnen durch Auflösung des modifizierten Chitosans und Polyethylenoxids (PEO) in einer wässrigen Lösung von Essigsäure,
- Anwendung der auf diese Weise vorbereiteten zu vernetzender Lösung auf eine entsprechend gewählte Trägersubstanz,
- Vernetzen des Materials der Nanofiber-Membrane.

3. Das Verfahren nach Anspruch 2, **gekennzeichnet durch** die folgenden Schritte:
- Mischungsvorbereitung des Chitosan-Monomers mit einer -NH₂-Gruppe bei 80% Deacetylation gelöst in einem aquatischen Medium im Verhältnis von 5 g Chitosan zu 10 ml Wasser mit Glycidylmethacrylat (GMA) im Verhältnis von 2 mol Chitosan zu 1 mol GMA und Rühren des Gemisches während 24 Stunden,
- Filtration, Durchwaschen mit H₂O und Trocknen des Gemisches ohne Lichteinfall während zwei Tagen,
- Stoßen des Endprodukts, der festen Phase des in saurem Milieu löslichen modifizierten Chitosans, zu Pulver,
- nachfolgende Vorbereitung eines Gemisches zum Verspinnen durch Auflösung des modifizierten Chitosans und PEO in einer wässrigen Lösung der Essigsäure in einem Volumenverhältnis des Wasser zur Essigsäure 2:1, sodass die resultierende Lösung 2 %-Gew. bis 10 %-Gew. Chitosan und 0,1 %-Gew. bis 0,3 %-Gew. PEO enthält,
- Anwendung der auf diese Weise vorbereiteten zu vernetzender Lösung auf eine entsprechend gewählte Trägersubstanz,
- Vernetzen des Materials der Nanofiber-Membrane.

4. Das Verfahren nach Anspruch 2 oder 3, **dadurch gekennzeichnet, dass** das Vernetzen mittels UV-Bestrahlung der Nanofiber-Membrane vollzieht wird.

5. Das Verfahren nach Anspruch 2 oder 3, **gekennzeichnet durch** die folgenden Schritte:
- Zugabe der Gelatine in die Lösung des modifizierten Chitosans und PEO, sodass die resultierende Lösung 4 %-Gew. bis 9 %-Gew. Chitosan, 0,1 %-Gew. bis 0,3 %-Gew. PEO und 0,5 %-Gew. bis 5,0 %-Gew. Gelatine enthält,
- Vernetzen der Nanofiber-Membrane mittels UV-Strahlung.

6. Das Verfahren nach Anspruch 5, **dadurch gekennzeichnet, dass** das Vernetzen mittels Aussetzung der Nanofiber-Membrane der Temperatur von 130°C während ungefähr 10 Minuten vollzieht wird.

7. Das Verfahren nach einem der Ansprüche 2 bis 6, **dadurch gekennzeichnet, dass** in das Gemisch zum Verspinnen als Vernetztmittel Tetramethylen-Glykol-Dimethakrylat (TEGMA) hinzufügen wird.

8. Das Verfahren nach einem der Ansprüche 2 bis 7, **dadurch gekennzeichnet, dass** die Nanofiber-Membrane durch Laminierung mit einer anderen Schicht eines Textilträgermaterials mittels eines Klebers oder durch eine andere Technologie verbindet wird.

9. Das Verfahren nach Anspruch 8, **dadurch gekennzeichnet, dass** die bei der Herstellung der Nanofiber benutzte Trägersubstanz im Laufe der Laminierung oder vor der Anwendung des Composits entfernt wird.

10. Das Verfahren nach Anspruch 9, **dadurch gekennzeichnet, dass** die Nanofiber-Membrane mit einer Schutzverpackung versehen ist, die vor Anlegen an die Wunde abgenommen wird.

## Revendications

1. Nanofibres en chitosane ou un mélange de chitosane/gélatine, dans lequel le chitosane est chimiquement modifié par des groupes méthacrylate.

2. Une méthode de production d'une couche de nanofibres par des nanofibres de chitosane selon la revendication 1 dans une solution d'oxyde de polyéthylène (PEO) conçue pour un composite textile pour pansement, **caractérisé en des** pas suivants :
- mélange d'un monomère de chitosane avec un agent contenant du méthacrylate dans un milieu aqueux,
- soumettre le mélange à la filtration, le laver avec H₂O et le sécher sans accès à la lumière,
- broyer le produit résultant en poudre, ce qu'est un chitosane modifié en phase solide soluble,
- ensuite préparer un mélange de réticulation en dissolvant le chitosane modifié et l'oxyde de polyéthylène (PEO) dans une solution aqueuse d'acide acétique,
- appliquer la solution de réticulation préparée de cette manière sur un substrat convenablement sélectionné,
- réticulation du matériau dans la couche nanofibre.

3. Une méthode selon la revendication 2, **caractérisé en ce que** des pas suivants :
- mélanger un monomère de chitosane avec un groupe -NH₂ à 80% de désacétylation dissous dans un environnement aqueux dans un rapport de 5 g de chitosane à 10 ml d'eau avec du méthacrylate de glycidyle (GMA) dans un rapport de 2 mol de chitosane à 1 mol de GMA, et mélanger la mixtion pendant 24 heures,
- soumettre la mixtion à la filtration, le laver par H₂O et le sécher pendant deux jours sans accès à la lumière,
- broyer le produit résultant en poudre, ce qu'est la phase solide dans un milieu aqueux acide du chitosane modifié soluble,
- préparer ultérieurement un mélange de réticulation en dissolvant le chitosane modifié et le PEO dans une solution aqueuse d'acide acétique au rapport volumique de l'eau à l'acide acétique 2 à 1 de telle sorte que la solution résultante contienne 2 % en poids à 10 % en poids de chitosane et 0,1 % en poids à 0,3 % en poids d'OEP,
- appliquer la solution de réticulation préparée de cette manière sur un substrat convenablement sélectionné,
- réticulation du matériau dans la couche nanofibre.

4. Une méthode selon la revendication 2 ou 3, **caractérisé en ce que** la réticulation est réalisée en exposant la couche de nanofibre au rayonnement UV.

5. Une méthode selon la revendication 2 ou 3, **caractérisé en ce que** des pas suivants :
- l'ajout de la gélatine à la solution de chitosane et de PEO modifiés de telle sorte que la solution résultante contienne 4 % en poids à 9 % en poids de chitosane, 0,1 % en poids à 0,3 % en poids de PEO et 0,5 % en poids à 5,0 % en poids de gélatine,
- réticulation du matériau dans la couche nanofibre en l'exposant aux rayons UV.

6. Une méthode selon la revendication 5, **caractérisé en ce que** la réticulation est réalisée en exposant la couche de nanofibre à la température 130°C pendant une période d'environ 10 min.

7. Une méthode selon les revendications 2 à 6, **caractérisé en ce que** l'agent de réticulation tétraméthylène glycol diméthacrylate (TEGMA) est ajouté au mélange.

8. Une méthode selon les revendications 2 à 7, **caractérisé en ce que**, la couche de nanofibres est reliée, par un procédé de laminage, à une autre couche composite textile à l'aide d'adhésifs ou des technologies non adhésives.

9. Une méthode selon la revendication 8, **caractérisé en ce que** le substrat utilisé dans la production de nanofibres est éliminé pendant le processus de laminage ou avant l'application du composite.

10. Une méthode selon la revendication 9, **caractérisé en ce que** la couche de nanofibres est munie d'une enveloppe protectrice qui est retirée avant l'application sur la blessure.
